# EUROPEAN PATENT APPLICATION

(11) **EP 4 628 885 A1**
(43) Date of publication of application: **08.10.2025**
(21) Application number: 24168446.3
(22) Date of filing: 04.04.2024
(51) Int. Cl.: G01N 27/411

(54) **ELEMENT TO MEASURE THE OXYGEN CONTENT OF MOLTEN METALS**

(71) Applicant: Heraeus Electro-Nite International N.V., 3530 Houthalen (BE)
(72) Inventor: Neyens, Guido, 3530 Houthalen (BE); Bex, Gert-Jan, 3530 Houthalen (BE)
(74) Representative: Heraeus IP

(57) **Abstract**

Oxygen detecting element, comprising a coated pin. The coated pin comprises an electrically conductive core with a tapered section towards one end. The coating comprises a two-layered coating structure on a tip portion and a three-layered coating structure on a main portion of the electrically conductive core, wherein the tapered section has the same length or is longer than the tip portion. The invention further relates to an immersion sensor comprising the oxygen detecting element and a method for measuring the oxygen content of a metal melt with such an oxygen detecting element.

## Description

The invention relates to an oxygen detecting element comprising a coated pin. The coated pin comprises an electrically conductive core with a tapered section towards one end. The coating comprises a two-layered coating structure on a tip portion and a three-layered coating structure on a main portion of the electrically conductive core, wherein the tapered section has the same length or is longer than the tip portion. The invention further relates to an immersion sensor comprising the oxygen detecting element and a method for measuring the oxygen content of a metal melt with such an oxygen detecting element.

During metallurgical processes, the oxygen activity of a metal melt is one of the parameters which needs to be monitored. Determining the oxygen activity is typically done with an electrochemical sensor, comprising a solid electrolyte material, a reference material, and an electrode. The electromotive force (EMF) generated by the difference between the constant oxygen partial pressure given by the reference material and the oxygen partial pressure in the molten metal is then monitored and related to the activity or concentration of oxygen in the liquid metal. Many electrochemical sensors for the testing of such melts have shortcomings as slow response rates, high failure rates, poor reproducibility, and low sensitivity.

A type of oxygen sensor is the needle sensor, which comprises a conductive wire which serves as an electrode with at least a solid electrolyte coating and a reference material coating. These sensors suffer from a slow response time or inadequate stability for the application in the demanding environment of molten metals. An electro-chemical equilibrium between the molten metal and the oxygen sensor is necessary for an exact measurement of the EMF-value. However, an electro-chemical equilibrium only occurs if there is a thermal equilibrium between the immersion probe and its surroundings.

In order to obtain accurate measurement values, the temperature of the metal bath needs to be determined in parallel to the oxygen activity. The response time of the oxygen sensing device should ideally be faster than the response time of the temperature sensor. Mostly, thermocouples with a response time of 3 - 6 seconds are employed for this purpose.

JP S6179156 A discloses a needle-type oxygen concentration detecting element with a metallic wire, which comprises a coating with a conical shape to lower the response time of the device.

US 5332449 A also discloses a needle sensor. The sensing device comprises a conductive wire with a uniform thickness, which is coated with an electrolyte material, a reference material and a refractory material. In order to improve the thermal responsiveness of the device, it is suggested to narrow the diameter of the conductive pin in the area without the functional coating. This configuration has been found to weaken the mechanical stability of the device.

The present invention overcomes at least parts of the problems identified in the prior art. In particular, it was an objective of the present invention to provide an oxygen detecting element with a fast response time and a high mechanical strength. A further aspect was to provide an oxygen detecting element which can be produced reliably and in a fast and efficient manner. It was an additional objective to provide an oxygen detecting element with a low-cost design.

In a different aspect, it was an objective to provide an immersion sensor with the inventive oxygen detecting element.

In a further aspect, it was an objective to provide a method for measuring the oxygen content of a metal melt with the inventive oxygen detecting element.

The present invention provides an oxygen detecting element comprising a coated pin. The coated pin comprises an electrically conductive core which extends longitudinally from a main portion to a tip portion and the tip portion ends in a tip end.

The tip portion is covered by a tip coating structure (CS-T) which comprises
(i) an inner coating which covers and is in direct contact with at least a part of the tip portion, the inner coating comprising a reference material, and
(ii) an outer coating which covers and is in direct contact with at least a part of the inner coating, the outer coating comprising an electrolyte material.

The main portion is covered by a main coating structure (CS-M) and comprises
(i) an inner coating which covers and is in direct contact with at least a part of the main portion, the inner coating comprising a reference material,
(ii) an intermediate coating which covers and is in direct contact with the inner coating, the intermediate coating comprising a refractory material, and
(iii) an outer coating which covers and is in direct contact with at least a part of the intermediate coating, the outer coating comprising an electrolyte material.

The electrically conductive core comprises a tapered section, which is a section comprising a cross-section which is tapered in a longitudinal direction towards the tip end. The tapered section has a length L_{TS} and the tip portion has a length L_{TP}. The oxygen detecting element is characterized in that the tapered section has the same length or is longer than the tip portion (L_{TS} ≥ L_{TP}).

The tapered tip portion with the coating of a reference material and an electrolyte material functions as the measuring zone in this configuration. Surprisingly, it has been found that a measuring zone with a minimized diameter, in other words a measuring zone with a tapered cross-sectional profile, results in an oxygen detecting element with a shortened response time which still provides the required mechanical strength for the intended application. While the typical response times for such sensors is in the region of 6 to 10 s, the oxygen detecting element according to the present invention shows significantly shorter response times in the range of 1 to 4 s.

For certain applications the oxygen detecting element is mounted on an immersion device to bring it in contact with the molten metal, typically comprising at least a carrier tube. These carrier tubes need to withstand the circumstances of immersion prior to their decomposition at least long enough for the measurement to be conducted, which is mostly realized by the provision of a certain amount of material. A faster response time of the oxygen detecting element allows a reduced use of material, resulting in a reduction of the costs for the immersion device. For example, the thickness of a carrier tube made of cardboard can be reduced approximately by 1 mm in diameter for every second the response time is shortened.

The object of the present invention is an oxygen detecting element comprising a coated pin. The coated pin comprises an electrically conductive core and several coating layers in at least two coating structures superimposed on the surface of the conductive core. The different coating structures together form the coating of the coated pin.

Examples for suitable materials for the electrically conductive core are molybdenum (Mo) and tungsten (W), especially due to their thermal properties. Preferably, the material of the conductive core comprises Mo, even more preferred the electrically conductive core consists of Mo except for unavoidable impurities. The cross-sectional area of the electrically conductive core can have any shape, preferably it is round, oval or elliptical. It is advantageous for short response times, that the maximum cross-sectional area of the electrically conductive core is in the range between 0,1 and 3 mm², especially between 0,3 and 1,5 mm².

The electrically conductive core extends longitudinally from a main portion to a tip portion, wherein the tip portion ends in a tip end. The axis extending from the main portion to the tip portion is referred to as the longitudinal axis of the electrically conductive core and/or the coated pin throughout this application. The electrically conductive core may comprise further portions. The length of the electrically conductive core is preferably in the range of 40 to 100 mm. The length of the electrically conductive core is to be understood as the length from the tip end to the other end.

The electrically conductive core comprises a tapered section, the tapered section being a section comprising a cross-section which is tapered in a longitudinal direction towards the tip end. In other words, the electrically conductive core comprises the tip end, which is a tapered end and another end, and the cross-sectional area of the electrically conductive core is smaller at the tapered end. If not otherwise defined, a cross-section or cross-sectional area is the cross-section or cross-sectional area perpendicular to the longitudinal axis of the electrically conductive core along its length.

It is to be understood, that the tip portion and the tapered section overlap at least partially. In other words, the tapered section comprises the tip portion.

The tapered section can extend over the whole length of the electrically conductive core. It can also extend over only a part of its length; in such cases the electrically conductive core comprises at least two sections: the tapered section and a section with a constant diameter and cross-sectional area.

The tapered section has a length L_{TS}. Preferably, the tapered section extends over at least 10 % of the length of the electrically conductive core, more preferably over at least 20 %, even more preferred over at least 30 %. The length of the tapered section typically is in the range of 4 to 30 mm, preferably in the range of 8 to 20 mm.

The tapered section can have the same or a different cross-sectional shape as optionally present additional sections, for example the tapered section may have a rectangular cross-section and the other section may have a round, oval or elliptical shape.

The shape of the tapered section may vary, especially depending on the production method of the electrically conductive core. The tapered section may have a radial-symmetrical geometry in relation to the central longitudinal axis of the electrically conductive core, in such cases it may for example be conical or frustoconical shaped. The tapered section may also have a geometry which is not radial symmetric in relation to the central longitudinal axis, in such cases it may for example be conical, frustoconical, prismatic or pyramidal shaped.

Preferably, the tapered section has a conical shape, in other words it has a round or oval cross-section and ends in a round or oval shaped tapered end.

The degree of tapering of the tapered section may be described by a taper angle, this angle is to be understood as the angle between the two tangents adjacent to the surface of the tapered section in the plane of the maximum cross-sectional area of the tapered section along its longitudinal axis. It has been shown to be favorable when the taper angle is smaller than 40°, even more preferred smaller than 30°, most preferred smaller than 20°. The taper angle may be in the range between 1 to 40°, preferably in the range between 3 to 30°, even more preferred in the range between 5 to 20°.

In preferred embodiments, the electrically conductive core is needle shaped, in other words it comprises a round-shaped cross-sectional area over its whole length, a tapered section with a conical shape and a tapered end with a round-shaped cross-section.

The tip end may have different shapes, for example it may be dome-shaped or flat.

Preferably, the cross-sectional area of the tip end is smaller than 40 % of the maximum cross-sectional area of the electrically conductive core, more preferably smaller than 30 %, even more preferred smaller than 20 %. For example, the cross-sectional area of the pin end may be in the range of 0,5 % to 40 % of the maximum cross-sectional area, more preferably in the range of 2 % to 30 %, most preferred in the range of 5 % to 20 %. It has been found to be favorable for short response-times that the cross-sectional area of the pin end is between 0,01 and 1 mm², preferred between 0,02 and 0,5 mm², more preferred between 0,05 and 0,2 mm².

The coated pin comprises a coating, which comprises at least a tip coating structure and a main coating structure.

The coating may have any cross-sectional shape perpendicular to the longitudinal axis of the conductive core, preferably, the cross-section has a round, oval or elliptical shape, even more preferred the cross-section has an elliptical shape. In other words, the coating may have a constant thickness perpendicular to the longitudinal axis of the conductive core, the thickness may also vary.

The geometry of the cross-sectional area of the coating can be defined by two intersecting axis which meet at an intersection point (IP): a major axis is aligned with the maximum diameter of the cross-sectional area and has a length D_{MJ}, which corresponds to the maximum diameter of the cross-sectional area. A minor axis is perpendicular to the major axis and has a length D_{MI}. The minor axis is positioned along the largest diameter perpendicular to the major axis. In case of a circular cross-sectional area, the major axis and the minor axis are of equal length. The intersection point IP may be considered as the center of the cross-sectional area.

The center of the conductive core may be positioned centrally in the coating, in other words, the center of the conductive core and the intersection point IP of the cross-sectional area of the coating may coincide. The center of the electrically conductive core may also be positioned eccentrically in the coating, in such cases the center of the conductive core and the intersection point IP of the cross-sectional area of the coating do not coincide. An eccentric position of the center of the conductive core in the coating has surprisingly been found to have a positive effect on the response time of the oxygen detecting element and the stability of the coating.

In an eccentric configuration, the center of the conductive core is typically positioned with an offset to the intersection point IP along the major axis of the cross-section of the coating, thus, the coating comprises two thicknesses along the major axis, a smaller thickness Ts and a larger thickness T_{MAX}, which corresponds to the maximal thickness of the coating structure in the cross-sectional area. It is to be understood, that the thickness of the coating and the maximal thickness of the coating may vary along the length of the coated pin.

In preferred embodiments, the maximal thickness T_{MAX} is at least 5 % larger than the smaller thickness Ts, more preferred at least 8 % larger. For example, the maximal thickness T_{MAX} is 5 to 20 % larger than the smaller thickness Ts, more preferred 8 to 15 % larger.

The tip portion of the electrically conductive core is covered by a tip coating structure (CS-T). It is to be understood, that the tip portion of the electrically conductive core is characterized in that it is covered by the tip coating structure. The tip coating structure also covers the tip end, in other words the tip coating structure encloses the tip end.

The tip portion and the tip coating structure build the measuring section of the coated pin. It is to be understood, that the measuring section of the coated pin has a tapered cross-section towards the tip end. In other words, not only the electrically conductive core has a tapered end, but this tapering is also present for the whole coated pin.

The tip portion has a length L_{TP}. Preferably, the tip portion extends over not more than 10 % of the length of the electrically conductive core, more preferably over not more than 5 %, even more preferred over not more than 1 %. The length of the tip portion may be in the range of 0,1 to 10 mm, more preferred in the range of 1 to 8 mm.

The oxygen detecting element of the present invention is characterized in that the length of the tapered section of the electrically conductive core has the same length or is longer than the length of the tip portion of the electrically conductive core (L_{TS} ≥ L_{TP}). Since the measuring zone is to be found in the tapered section of the coated pin, such a configuration allows a fast heating of the measuring zone and results in a short response time of the oxygen detecting element.

The length of the tip portion L_{TP} is preferably at least 20 % of the length of the tapered section L_{TS} of the electrically conductive core, more preferably at least 30 %, even more preferred at least 50 %.

The tip coating structure may have any cross-sectional shape perpendicular to the longitudinal axis of the conductive core, preferably, the cross-section has a round, oval or elliptical shape, even more preferred the cross-sectional has an elliptical shape. In other words, the tip coating structure may have a constant thickness perpendicular to the longitudinal axis of the conductive core, the thickness may also vary.

The geometry of the cross-sectional area of the tip coating structure can be defined by two intersecting axis which meet at an intersection point (IP): A major axis is aligned with the maximum diameter of the cross-sectional area and has a length D_{MJ}-T, which corresponds to the maximum diameter of the cross-sectional area. A minor axis is perpendicular to the major axis and has a length D_{MI}-T. The minor axis is positioned along the largest diameter perpendicular to the major axis.

The center of the conductive core may be positioned centrally in the tip coating structure, in other words, the center of the conductive core and the intersection point IP of the cross-sectional area of the tip coating structure may coincide. The center of the electrically conductive core may also be positioned eccentrically in the tip coating structure, in such cases the center of the conductive core and the intersection point IP of the cross-sectional area of the tip coating structure do not coincide. An eccentric position of the center of the conductive core in the tip coating structure has surprisingly been found to shorten the response time of the oxygen detecting element.

In an eccentric configuration, the center of the conductive core is typically positioned with an offset to the intersection point IP along the major axis of the tip coating structure, thus, the tip coating-structure comprises two thicknesses along the major axis, a smaller thickness Ts-T and a larger thickness T_{MAX}-T, which corresponds to the maximal thickness of the coating structure in the cross-sectional area.

In preferred embodiments, the maximal thickness T_{MAX}-T is at least 5 % larger than the smaller thickness Ts-T, more preferred at least 8 % larger. For example, the maximal thickness T_{MAX}-T is 5 to 20 % larger than the smaller thickness Ts-T, more preferred 8 to 15 % larger.

In preferred embodiments, the tip coating structure has a minimal thickness of at least 0,06 mm, more preferred of at least 0,1 mm. The minimal thickness of any of the coating structures or coating layers is to be understood as the smallest thickness of the respective structure or layer perpendicular to the longitudinal axis of the conductive core. For example, the tip coating structure can have a thickness between 0,06 - 0,5 mm, more preferred between 0,1 - 0,4 mm. The thickness of the tip coating structure may be uniform along the length of the coated pin, the thickness may also vary.

The tip coating structure (CS-T) comprises and inner coating and an outer coating. The inner coating covers and is in direct contact with at least a part of the tip portion and the outer coating covers and is in direct contact with at least a part of the inner coating. In other words, no coating layer is positioned between the inner and the outer coating. Additional coating layers may be present on top of the outer coating.

The inner coating of the tip coating structure comprises a reference material. Preferably, the reference material comprises a metal-metal oxide mixture, for example a mixture of chromium and chromium dioxide (Cr-Cr₂O₃) or molybdenum and molybdenum oxide (Mo-MoO₂). The inner coating of the tip coating structure preferably has a thickness of at least 0,01 mm, more preferably of at least 0,03 mm, even more preferred of at least 0,05 mm. In this context the term "thickness" or "coating thickness" refers to the minimal thickness of the coating layer perpendicular to the longitudinal axis of the electrically conductive core. For example, the inner coating can have a thickness between 0,01 to 0,3 mm, more preferred between 0,03 to 0,2 mm. The thickness of the reference material coating may be uniform along the length of the coated pin, the thickness may also vary.

The outer coating of the tip coating structure comprises an electrolyte material. The electrolyte material is preferably a solid material with oxygen ion conducting activity. Preferably, the electrolyte material comprises zirconium oxide (zirconia, ZrO₂) or a stabilized zirconium oxide (stabilized zirconia). As known to the skilled person, a stabilized zirconium oxide comprises at least one kind of oxide such as magnesia (MgO), calcium oxide (CaO), yttria (Y₂O₃), ceria (CeO₂) or scandia (Sc₂O₃) solid-dissolved as a stabilizer in zirconia. The outer coating preferably has at least a thickness of 0,05 mm, more preferably of at least 0,1 mm, even more preferred of at least 0,15 mm. For example, the outer coating can have a thickness between 0,05 to 0,5 mm, more preferred between 0,1 to 0,4 mm. The thickness of the electrolyte material coating may be uniform along the length of the coated pin, the thickness may also vary.

The main portion of the electrically conductive core is covered by a main coating structure (CS-M). It is to be understood, that the main portion of the electrically conductive core is characterized in that it is covered by the main coating structure.

The main portion and the tapered section may overlap at least partially or completely. In other words, the tapered section may comprise at least parts of the main portion. It is also possible, that the main portion does not overlap with the tapered section.

The main portion has a length L_{MP}. Preferably, the main portion extends over more than 30 % of the length of the electrically conductive core, more preferably over more than 40 %, even more preferred over more than 50 %. The length of the main portion is typically in the range of 5 to 50 mm, preferably in the range of 10 to 40 mm.

The main coating structure may have any cross-sectional shape perpendicular to the longitudinal axis of the electrically conductive core, preferably, the cross-section has a round, oval or elliptical shape, even more preferred the cross-section has an elliptical shape. In other words, the main coating structure may have a constant thickness perpendicular to the longitudinal axis of the conductive core, the thickness may also vary.

The cross-sectional area of the main coating structure may be described by analogue parameters as the cross-sectional area of the tip coating structure: a major axis with a length D_{MJ}-M, which corresponds to the maximal diameter of the cross-sectional area and a minor axis perpendicular to the major axis with a length D_{MI}-M.

The center of the conductive core may be positioned centrally in the main coating structure, in other words, the center of the conductive core may and the intersection point IP of the cross-sectional area of the main coating structure may coincide. The center of the electrically conductive core may also be positioned eccentrically in the main coating structure, in such cases the center of the conductive core and the intersection point IP of the cross-sectional area of the main coating structure do not coincide. In an eccentric configuration, the center of the conductive core is typically positioned with an offset to the intersection point IP along the major axis of the main coating structure, thus, the main coating-structure comprises two thicknesses along the major axis, a smaller thickness Ts-M and a larger thickness T_{MAX}-M, wherein T_{MAX}-M corresponds to the maximal thickness of the main coating structure in the cross-sectional area.

In preferred embodiments, the maximal thickness T_{MAX}-M is at least 5 % larger than the smaller thickness Ts-M, more preferred at least 8 % larger. For example, the maximal thickness T_{MAX}-M is 5 to 20 % larger than the smaller thickness Ts-M, more preferred 8 to 15 % larger.

In preferred embodiments, the main coating structure has a minimal thickness of at least 0,07 mm, more preferred of at least 0,12 mm. For example, the main coating structure can have a thickness between 0,07 to 0,8 mm, more preferred between 0,12 to 0,6 mm. The thickness of the main coating structure may be uniform along the length of the coated pin, the thickness may also vary.

In preferred embodiments, the main coating structure has a larger minimal thickness than the tip coating structure. Preferably, the main coating structure has a larger minimal diameter than the tip coating structure.

The main coating structure (CS-M) comprises an inner coating, an intermediate coating and an outer coating. The inner coating covers and is in direct contact with at least a part of the main portion of the electrically conductive core, the intermediate coating covers and is in direct contact with the inner coating and the outer coating covers and is in direct contact with at least a part of the intermediate coating. Additional coating layers may be present on top of the outer coating.

The inner coating of the main coating structure comprises a reference material. The reference material of the main coating structure may be the same as the reference material of the tip coating structure.

The inner coating of the main coating structure preferably has a thickness of at least 0,01 mm, more preferably of at least 0,03 mm, even more preferred of at least 0,05 mm. For example, the inner coating can have a thickness between 0,01 to 0,3 mm, more preferred between 0,03 to 0,2 mm.

Preferably, the inner coating of the main coating structure and the inner coating of the tip coating structure are sections of an inner coating layer, in other words they build a single coating layer over at least the main portion and the tip portion of the electrically conductive core. The thickness of the inner coating layer may be uniform along the length of the coated pin, the thickness may also vary.

The intermediate coating of the main coating structure comprises a refractory material. Preferably, the refractory material comprises an oxidic material, for example an aluminum oxide like Al₂O₃, a magnesium oxide, a titanium oxide or mixtures thereof. Preferably, the refractory material comprises aluminum oxide. The intermediate coating preferably has a thickness of at least 0,01 mm, more preferably of at least 0,03 mm, even more preferred of at least 0,05 mm. For example, the intermediate coating can have a thickness between 0,01 to 0,3 mm, more preferred between 0,03 to 0,2 mm.

The outer coating of the main coating structure comprises an electrolyte material. The electrolyte material of the main coating structure may be the same as the electrolyte material of the tip coating structure.

The outer coating of the main coating structure preferably has a thickness of at least 0,05 mm, more preferably of at least 0,1 mm, even more preferred of at least 0,15 mm. For example, the outer coating can have a thickness between 0,05 to 0,5 mm, more preferred between 0,1 to 0,4 mm.

Preferably, the outer coating of the main coating structure and the outer coating of the tip coating structure are sections of an outer coating layer, in other words they build a single coating layer over at least the main portion and the tip portion of the electrically conductive core. The thickness of the outer coating layer may be uniform along the length of the coated pin, the thickness may also vary.

The maximum cross sectional-area of the coated pin is typically located within the main portion. The cross-sectional area of the coated pin is to be understood as the combined cross-sectional area of the electrically conductive core and the surrounding coating layers. The maximum cross sectional-area of the coated pin may be in the range between 0,5 and 7 mm², especially between 1,0 and 6 mm².

Preferably, the cross-sectional area of the coated pin end of the electrically conductive core is smaller than 40 % of the maximum cross-sectional area of the coated pin, more preferably smaller than 30 %, even more preferred smaller than 20 %. The coated pin end is to be understood as the coated end and the tip coating structure. A ratio of the cross-sections in this range results in a coated pin with a fast response time with a simultaneous sufficient stability. For example, the cross-sectional area of the coated pin end may be in the range of 0,5 % to 40 % of the maximum cross-sectional area of the coated pin, more preferably in the range of 2 % to 30 %, most preferred in the range of 5 % to 20 %. It has been found to be favorable for short response-times that the cross-sectional area of the coated pin end is between 0,1 and 2,5 mm², especially between 0,5 and 1,5 mm².

The electrically conductive core may comprise one or more portions which are covered with further coating structures and/or portions which are not coated.

In preferred embodiments, the electrically conductive core comprises a third portion. In such cases, the electrically conductive core extends longitudinally from the third portion towards the main portion to the tip portion. The third portion is covered with a third coating structure (CS-3) which may comprise an intermediate coating and an outer coating. The intermediate coating covers and is in direct contact with at least a part of the third portion of the electrically conductive core and the outer coating covers and is in direct contact with at least a part of the intermediate coating. In other words, no coating layer is between the intermediate and the outer coating. Additional coating layers may be present on top of the outer coating.

The third portion and the tapered section of the electrically conductive core may overlap at least partially or completely. In other words, the tapered section may comprise at least parts of the third portion. It is also possible, that the third portion does not overlap with the tapered section.

The cross-sectional area of the third coating structure may be described by analogue parameters as the cross-sectional area of the tip coating structure and the main coating structure: a major axis with a length D_{MJ}-3, which corresponds to the maximal diameter of the cross-sectional area and a minor axis perpendicular to the major axis with a length D_{MI}-3.

The center of the conductive core may be positioned centrally in the third coating structure, in other words, the center of the conductive core may be aligned on the intersection point IP of the cross-sectional area of the main coating structure. The center of the electrically conductive core may also be positioned eccentrically in the third coating structure, in such cases the center of the conductive core and the intersection point IP of the cross-sectional area of the third coating structure do not coincide. In an eccentric configuration, the center of the conductive core is typically positioned with an offset to the intersection point IP along the major axis of the third coating structure, thus, the third coating-structure comprises two thicknesses along the major axis, a smaller thickness Ts-3 and a larger thickness T_{MAX}-3, which corresponds to the maximal thickness of the main coating structure in the cross-sectional area.

In preferred embodiments, the maximal thickness T_{MAX}-3 is at least 5 % larger than the smaller thickness Ts, more preferred at least 8 % larger. For example, the maximal thickness T_{MAX}-3 is 5 to 20 % larger than the smaller thickness Ts-3, more preferred 8 to 15 % larger.

In preferred embodiments, the third coating structure has a minimal thickness of at least 0,06 mm, more preferred of at least 0,1 mm. For example, the third coating structure can have a thickness between 0,06 to 0,6 mm, more preferred between 0,1 to 0,5 mm. The thickness of the third coating structure may be uniform along the length of the coated pin, the thickness may also vary.

In preferred embodiments, the third coating structure has a smaller minimal thickness than the main coating structure. Preferably, the third coating structure has a smaller minimal diameter than the main coating structure.

The third portion has a length L_{3P}. Preferably, the third portion extends over not more than 10 % of the length of the electrically conductive core, more preferably over not more than 5 %, even more preferred over not more than 1 %. The length of the third portion section is typically in the range of 0,1 to 10 mm, preferably in the range of 1 to 8 mm.

The intermediate coating of the third coating structure may comprise a refractory material. The refractory material of the third coating structure may be the same as the refractory material of the main coating structure. The intermediate coating of the third coating structure preferably has at least a thickness of 0,01 mm, more preferably of at least 0,03 mm, even more preferred of at least 0,05 mm. For example, the intermediate coating can have a thickness between 0,01 to 0,3 mm, more preferred between 0,03 to 0,2 mm.

Preferably, the intermediate coating of the third coating structure and the intermediate coating of the main coating structure are sections of an intermediate coating layer, in other words they build a single coating layer over at least the third portion and the main portion of the electrically conductive core. The thickness of the intermediate coating layer may be uniform along the length of the coated pin, the thickness may also vary.

The outer coating of the third coating structure preferably comprises an electrolyte material. The electrolyte material of the third coating structure may be the same as the electrolyte material of the tip coating structure and/or of the main coating structure. The outer coating of the third coating structure preferably has a thickness of at least 0,05 mm, more preferably of at least 0,1 mm, even more preferred of at least 0,15 mm. For example, the outer coating can have a thickness between 0,05 to 0,5 mm, more preferred between 0,1 to 0,4 mm.

Preferably, the outer coating of the third coating structure, the outer coating of the main coating structure and the outer coating of the tip coating structure are sections of an outer coating layer, in other words they build a single coating layer over at least the third portion, the main portion and the tip portion of the electrically conductive core. The thickness of the outer coating layer may be uniform along the length of the coated pin, the thickness may also vary.

Preferably, the electrically conductive core comprises a mounting portion which is not coated. In such cases, the electrically conductive core extends longitudinally from the mounting portion towards the main portion to the tip portion. If a third portion is present, this is arranged between the mounting portion and the main portion.

The manufacturing of the inventive oxygen detecting element is typically conducted in a stepwise manner, in which the different coating layers are applied sequentially. The manufacturing may for example comprise the following steps:
(i) providing the electrically conductive core;
(ii) coating the pin portion and the main portion of the electrically conductive core with a reference material;
(iii) masking the pin portion of the electrically conductive core;
(iv) coating at least the main portion of the electrically conductive core with a refractory material;
(v) unmasking the pin portion of the electrically conductive core;
(vi) coating at least the pin portion and the main portion with an electrolyte material.

The coatings of the coating structures may be applied by techniques known to the skilled person, for example by chemical or physical vapor deposition processes (PVD, CVD), additive manufacturing methods as 3D printing, or spray processes like plasma or flame spraying. Especially spray processes produce uniform and dense coatings. A suitable method is for example disclosed in EP 0543081 A1.

During such a spray process, the object to be coated is laterally moved through a spray cone comprising the coating material, provided by a suitable source like a plasma or flame source, for example by means of a spray gun. In the present case, this object is the electrically conductive core. During the passage through the spray cone, the object is typically rotated to provide a homogeneous circumferential coating. To obtain a coating with a centrally positioned conductive core, this rotational movement may be centralized relative to the coating source, while for an eccentrical coating, this rotational movement may also be eccentrical relative to the source and the lateral movement.

In a second aspect, the present invention relates to an immersion sensor comprising an oxygen detecting element according to the invention.

The immersion sensor may comprise further components, such as a counter electrode, additional measuring means like means for temperature measurement, means for mounting the oxygen detecting element, means for signal transfer, means for protecting the oxygen detecting element and/or means for immersion. Further components of an immersion sensor are known to the skilled person and are for example disclosed in US 4964736 A.

Means for temperature measurement may for example be a thermocouple as known to the skilled person. Means for temperature measurement are not mandatory for a functional immersion sensor, in cases where the temperature is needed the temperature can for example also be derived by external means.

Means for mounting the oxygen detecting element may for example be a refractory mounting material in which the oxygen detecting element can be partly embedded, preferably only an uncoated mounting portion of the electrically conductive core are embedded in such a refractory material.

Means for immersion of the immersion sensor may for example be carrier tubes, preferably made from cardboard. Means for immersion are not mandatory for a functional immersion sensor; for example, when the immersion sensor is a drop sensor. Drop-sensors and their components are known to the skilled person and are for example disclosed in EP 0997716 A1.

In a third aspect, the present invention relates to a method for measuring the oxygen content of a metal melt with an oxygen detecting element according to the invention. As known to the skilled person, the method comprises the immersion of the oxygen detecting element in the respective metal melt.

The method may for example comprise the following steps:
(i) Providing the oxygen detecting element;
(ii) immersion the oxygen detecting element in a metal melt;
(iii) measuring the oxygen content in the metal melt.

The step of measuring the oxygen content in the metal melt may comprise the measurement of the oxygen activity of the metal melt and relating this measurement to the oxygen content.

In practice the metal melt is contacted with an oxygen detecting element in accordance with the invention and the electrochemical potential of the element is measured, preferably over time. The electrochemical activity is determined by the potential expressed from the cell through lead means to an analyzing unit. By relating the electrochemical potential thus determined to electrochemical potential, which is experienced for a series of standards, the content of the oxygen within the metal melt can be determined.

The following schematic drawings show aspects of the invention for improving the understanding of the invention in connection with some exemplary illustrations. It should be understood, however, that the invention is not limited to the precise arrangements and instrumentalities shown. The elements of the drawings are not necessarily to scale relative to each other. Like reference numerals designate corresponding similar parts. Herein
- Figure 1: shows different geometries of tip-ends of electrically conductive cores.
- Figure 2: shows schematic cross-sections of conductive cores suitable for the present invention.
- Figure 3: shows a schematic longitudinal cross-sectional view of an oxygen detecting element according to the invention.
- Figure 4: shows additional embodiments of the present invention.
- Figure 5: shows schematic transverse cross-sectional views of oxygen detecting elements.
- Figure 6: shows response characteristics of an oxygen detecting element according to the prior art in comparison with an inventive oxygen detecting element.

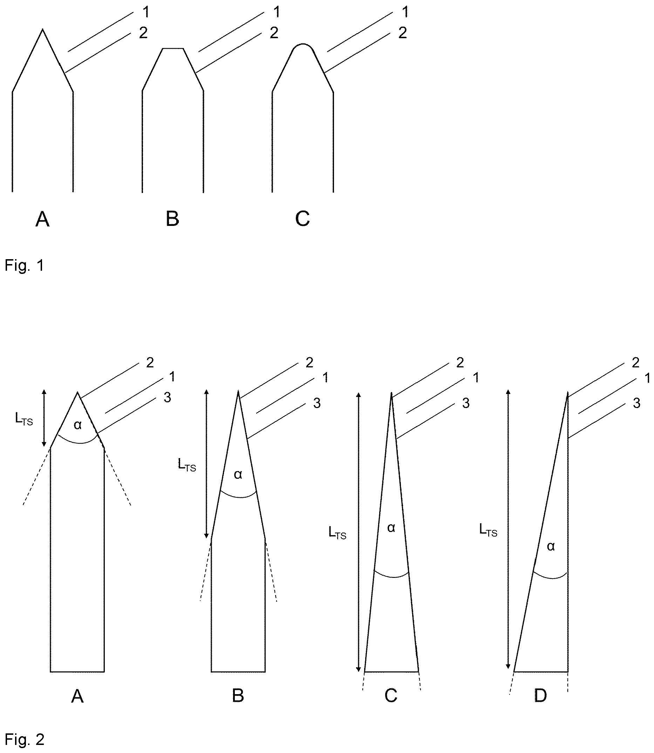

Figure 1 shows cross-sections of different geometries of tip-ends 2 of electrically conductive cores 1. The tip-end 2 in Fig. 1 A is needle shaped and ends in a sharp tip. The tip-end 2 shown in Fig. 1 B has a flattened needle shape, also referred to as a frustoconical shape. The tip-end 2 illustrated in Fig. 1 C comprises a dome-shaped needle tip.

Figure 2 shows schematic longitudinal cross-sections of conductive cores 1 suitable for the present invention. The pins 1 have different geometries with respect to the tapered section 3, its length (L_{TS}) and the degree of tapering at their ends. It is also illustrated where the degree of tapering is to be found, indicated by the taper angle α. The taper angle is determined by the angle between two tangents adjacent to the surface of the tapered section in the plane of the maximum cross-section of the tapered section along the longitudinal axis of the pin. These tangents are indicated by dashed lines. The pins 1 of Fig. 2 A - C have a central symmetrical geometry. Fig. 2 A shows a needle shaped conductive core 1 with a tapered section 3 which only extends over a part of the length of the pin. Fig. 2 B shows a similar geometry, whereas the tapered section 3 is longer and the taper angle α is smaller. The tapered section 3 of the conductive core 1 shown in Fig. 2 C extends over the whole length on the pin 1. The conductive core 1 of Fig. 2 D also has a tapered section 3 over its whole length, but the geometry is not central-symmetric.

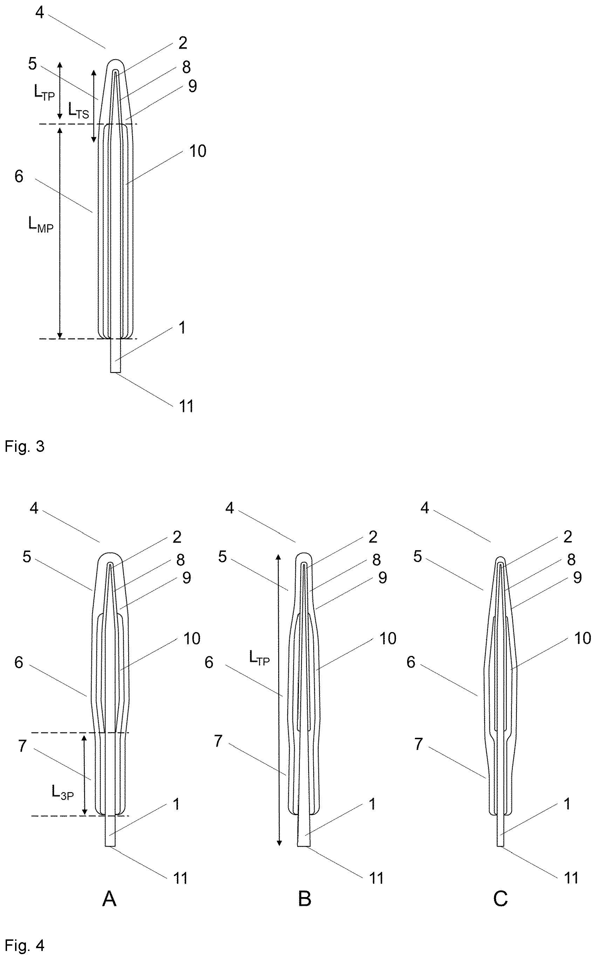

Figure 3 shows a schematic longitudinal cross-sectional view of an oxygen detecting element 4 according to the invention. The needle-shaped conductive core 1 has a coating structure with two portions: a first portion, the tip coating structure 5 (CS-T) covering the tip portion of the core 1 with a length L_{TP} and a second portion, the main coating structure 6 (CS-M), covering the main portion of the pin 1 with a length L_{MP}. The tip coating structure 5 and the tip end 2 provide the measuring zone of the sensor element 4 when it is in use. The tip coating structure 5 comprises a two-layered structure with a reference material coating 8 and an electrolyte material coating 9. Due to the reduced volume of the measuring zone on the tapered end of the conductive core, the oxygen detecting element has a fast response time. Surprisingly, such a geometry still allows for a robust oxygen detecting element.

The main coating structure 6 comprises a three-layered structure. Additional to the two layers on the tip coating structure (8, 9), it comprises a reference material layer 10, which extends between the reference material coating 8 and the electrolyte material coating 9.

In the displayed embodiment, the electrolyte material layer 9 and the reference material layer 8 extend over the whole length of the coating structure, while the refractory material layer 10 is only present over the length of the main portion L_{MP}.

The end of the conductive core 11 opposite the tip end 2 is not coated. Typically, this end is mounted in a suitable material, for example a refractory material, when the oxygen detecting element is mounted in a sensor assembly, it is therefore also referred to as mounting end.

The coating layers which cover the conductive core can be applied by means of a spray-process, e.g., plasma-spraying or flame-spraying, which produces a very uniform and dense coating. First, a reference material like chromium-chromium dioxide is applied to the conductive core. Subsequently, the part of the tapered section which shall become the measuring zone is masked and the refractory material, for example aluminum oxide, is applied to the unmasked part of the conductive core. Following removal of the masking, an electrolyte material like stabilized zirconium oxide is sprayed on.

The thicknesses of the different layers can be uniform along the length of the coated pin, but they can also vary, especially when a spraying process as described is applied to manufacture the sensing element. These different layers may have the same thickness, depending on the demand and application of the oxygen detecting element, they may also vary in shape and thickness.

Figure 4 shows additional embodiments of oxygen detecting elements 4 according to the present invention. In comparison to the embodiment of Fig. 3, the coating of the embodiment illustrated in Fig. 4 A comprises an additional third coating structure 7 on a third portion of the conductive core 1 with a length L_{3P}. L_{3P} is only indicated in Fig. 4 A for a better overview. The third coating structure 7 comprises a two-layered structure of the refractory material layer 10 and the electrolyte material layer 9. The two layers essentially have the same thickness. The conductive core 1 of the embodiment of Fig. 4 B is also needle shaped, but the tapering extends over the whole length of the pin. The coating structure also comprises three portions as in the embodiment of Fig. 4 A. The coating structure of the oxygen detecting element 4 of Fig. 4 C is similar to the coating structure of the embodiment as shown in Fig. 4 B. However, the outer shapes of the coating differ due to different thicknesses of the coating layers: While the refractory material layer 10 of Fig. 4 B has an essentially uniform thickness along the length of the oxygen detecting element 4, the thickness of this layer in Fig. 4 C comprises portions with varying thicknesses. Also, the electrolyte material layer 9 comprises portions with varying thickness, resulting in a coating structure with a barrel shape, i.e. a portion with a smaller diameter at the tip end 2, a maximal diameter in the main portion 6 and a third portion 7 with a tapering cross-sectional diameter.

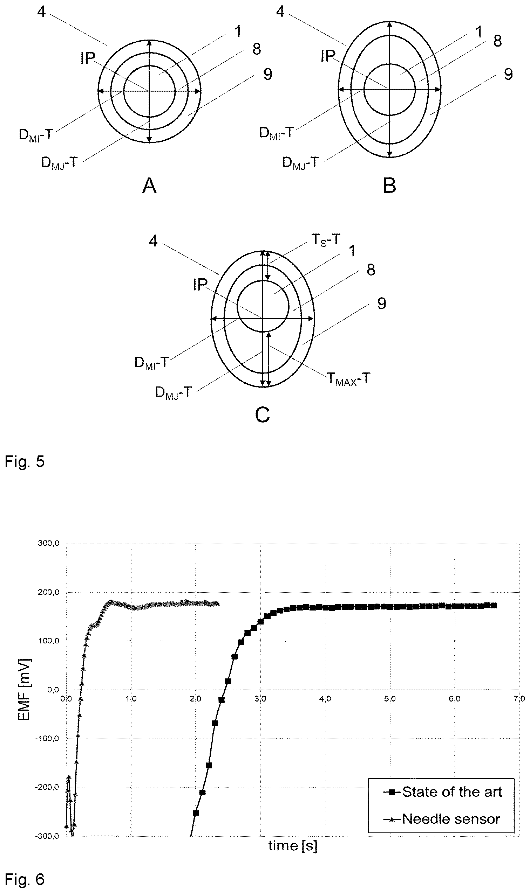

Figure 5 shows schematic transverse cross-sectional views of the measuring zone of oxygen detecting elements 4, i.e. in a plane perpendicular to the plane as shown in Fig. 1 - 4 in the area of the tip coating structure 5. The coating has a two-layered structure - a reference material coating 8 directly on the pin 1 and an electrolyte material coating 9 on top of the reference material coating 8. The coatings as shown in Fig. 5 A have a round shape with a uniform thickness. Accordingly, the length of the major axis D_{MJ}-T and of the minor axis D_{MI}-T of the cross-section are equal. The pin 1 is centrally positioned in the coating and the center of the pin is positioned on the intersection point IP of the two axes. The coatings in Fig. 5 B have an elliptical shape, in other words their circumferential thickness is not uniform. The pin 1 is centrally positioned in the coating structure. In Fig. 5 C a coating structure with an eccentrically positioned pin 1 is shown. The maximal thickness of the coating structure T_{MAX}-T as well as the smaller thickness Ts-T along the major axis are also indicated.

Figure 6 shows response characteristics of an oxygen detecting element according to the prior art in comparison with an inventive oxygen detecting element (needle sensor). Both sensors comprise a Mo pin with a three-layered coating structure. The pin of the state of the art sensor was a wire with a constant diameter of 1 mm. The pin of the inventive sensor was a Mo needle with a base diameter of 0,8 mm and a tip diameter of 0,2 mm. The tapered section has a length of 10 mm, of which the top 5 mm were coated with a two-layered structure comprising a 0,1 mm Cr/Cr₂O₃ mixture layer (the electrolyte material) and a 0,2 mm stabilized zirconia layer (the reference material). The zirconia layer extended over approximately the whole length of the Mo needle expect for a coating-free mounting section at the end. A 0,15 mm thick layer of Al₂O₃ (refractory material) was present in the lower part (in relation to the tip of the needle being the upper part) under the zirconia layer.

To obtain the curves, the respective detecting elements were immersed in a bath of molten steel and the detected electromotive force (EMF) was recorded over time. An electro-chemical equilibrium between the molten metal and the immersed sensor is necessary for an exact measurement of the EMF-value. Such an electro-chemical equilibrium only occurs if there is a thermal equilibrium between the sensor and its surroundings.

The shown data illustrate that the response time of the needle-shaped oxygen detecting element, i.e. the time until a constant signal could be achieved, is shortened significantly in comparison to a state of the art sensor without a tapered tip. Thus, a faster measurement can be achieved.

### Reference Signs

- 1: Conductive core
- 2: tip end of conductive core
- 3: tapered section of conductive core
- 4: oxygen detecting element
- 5: tip coating structure (CS-T)
- 6: main coating structure (CS-M)
- 7: third coating structure (CS-3)
- 8: reference material coating
- 9: electrolyte material coating
- 10: refractory material coating
- 11: mounting end of coated pin

- L_{TS}: length of tapered section
- L_{TP}: length of tip portion
- L_{MP}: length of main portion
- L_{3P}: length of third portion
- D_{MJ}-T: major axis of tip coating structure
- D_{MI}-T: minor axis of tip coating structure
- T_{MAX}-T: maximal thickness of tip coating structure
- Ts-T: smaller thickness of tip coating structure

- α: Taper angle

## Claims

1. An oxygen detecting element, comprising a coated pin,
the coated pin comprising an electrically conductive core which extends longitudinally from a main portion to a tip portion, wherein the tip portion ends in a tip end and wherein
(a) the tip portion is covered by a tip coating structure (CS-T),
wherein the tip coating structure (CS-T) comprises
(i) an inner coating which covers and is in direct contact with at least a part of the tip portion, the inner coating comprising a reference material, and
(ii) an outer coating which covers and is in direct contact with at least a part of the inner coating, the outer coating comprising an electrolyte material, and
(b) the main portion is covered by a main coating structure (CS-M),
wherein the main coating structure (CS-M) comprises
(i) an inner coating which covers and is in direct contact with at least a part of the main portion, the inner coating comprising a reference material,
(ii) an intermediate coating which covers and is in direct contact with the inner coating, the intermediate coating comprising a refractory material, and
(iii) an outer coating which covers and is in direct contact with at least a part of the intermediate coating, the outer coating comprising an electrolyte material,
wherein the electrically conductive core comprises a tapered section, the tapered section being a section comprising a cross-section which is tapered in a longitudinal direction towards the tip end,
wherein the tapered section has a length L_{TS} and the tip portion has a length L_{TP}
**characterized in that** L_{TS} ≥ L_{TP}.

2. An oxygen detecting element according to claim 1, wherein the tapered section extends over at least 10 % of the length of the electrically conductive core.

3. An oxygen detecting element according to claim 1 or 2, wherein the taper angle of the tapered section is smaller than 40°.

4. An oxygen detecting element according to any of the preceding claims, wherein the cross-sectional area of the tip end is smaller than 40 % of the maximum cross-sectional area of the electrically conductive core.

5. An oxygen detecting element according to any of the preceding claims, wherein the length of the tip portion L_{TP} is at least 20 % of the length of the tapered section L_{TS}.

6. An oxygen detecting element according to any of the preceding claims, wherein the coating of the coated pin has an elliptical shape and wherein the coating comprises at least the tip coating structure and the main coating structure.

7. An oxygen detecting element according to any of the preceding claims, wherein the center of the electrically conductive core is positioned eccentrically in the coating and wherein the coating comprises at least the tip coating structure and the main coating structure.

8. An oxygen detecting element according to any of the preceding claims, wherein the main portion extends over more than 30 % of the length of the electrically conductive core.

9. An oxygen detecting element according to any of the preceding claims, wherein the main coating structure has a larger minimal thickness than the tip coating structure.

10. An oxygen detecting element according to any of the preceding claims, wherein the tip coating structure has a minimal thickness of at least 0,06 mm.

11. An oxygen detecting element according to any of the preceding claims, wherein the main coating structure has a minimal thickness of at least 0,07 mm.

12. An oxygen detecting element according to any of the preceding claims, wherein the maximum cross sectional-area of the coated pin is located within the main portion.

13. An oxygen detecting element according to any of the preceding claims, wherein the electrically conductive core comprises a third portion and the third portion is covered with a third coating structure (CS-3) which comprises
(i) an intermediate coating which covers and is in direct contact with at least a part of the third portion of the electrically conductive core, the intermediate coating comprising a refractory material, and
(ii) an outer coating which covers and is in direct contact with at least a part of the intermediate coating, the outer coating comprising an electrolyte material.

14. Immersion sensor comprising an oxygen detecting element according to any of claims 1 - 13.

15. A method for measuring the oxygen content of a metal melt with an oxygen detecting element according to any of claims 1-13.
